# EUROPÄISCHE PATENTSCHRIFT

(11) **EP 3 833 875 B1**
(45) Veröffentlichungstag und Bekanntmachung des Hinweises auf die Patenterteilung: **26.07.2023**
(21) Anmeldenummer: 19725310.7
(22) Anmeldetag: 14.05.2019
(51) Int. Cl.: F04D 25/06, F04D 29/42

(54) **RADIALVENTILATOR**
RADIAL VENTILATOR
VENTILATEUR RADIAL

(30) Priorität: 23.11.2018 DE 102018129613; 23.11.2018 DE 102018129611; 23.11.2018 DE 102018129608
(43) Veröffentlichungstag der Anmeldung: 16.06.2021
(73) Patentinhaber: EBM-PAPST ST. GEORGEN GMBH & CO. KG, 78112 St. Georgen (DE)
(72) Erfinder: EHLERS, Volker, 78112 St. Georgen (DE); SCHAAKE, Katrin, 88662 Überlingen (DE)
(74) Vertreter: Staeger & Sperling Partnerschaftsgesellschaft mbB
(86) Internationale Anmeldenummer: PCT/EP2019/062393
(87) Internationale Veröffentlichungsnummer: WO 2020/104075

(56) Entgegenhaltungen:
- WO-A1-2016/169610
- DE-A1- 10 245 798
- DE-T5-112014 002 022

## Beschreibung

Die Erfindung betrifft einen Radialventilator ausgebildet als Hochdrehzahl- Radialventilator mit verbessertem Druckaufbau und Wirkungsgrad bei gleichzeitig geringerer Geräuschentwicklung.

Gattungsbildende Radialventilatoren sind aus dem Stand der Technik bekannt, beispielsweise aus dem Deutschen Gebrauchsmuster DE202018106694U1. Weiterer Stand der Technik im vorliegenden technischen Gebiet ist aus den Dokumenten WO 2016/169610 A1, DE 102 45 798 A1 und DE 11 2014 002 022 T5 bekannt.

Entsprechende Radialventilatoren haben grundsätzlich das Problem geringen Bauraums, jedoch soll gleichzeitig die Strömung möglichst optimal vom Ansaug zum Ausblas gefördert werden. Gute Strömungsverhältnisse verbessern den Wirkungsgrad, den Druckaufbau und die Akustik des Radialgebläses. Typischerweise weisen Radialventilatoren ein spiralartiges Gehäuse auf, welches die vom Radialrad abgegebene schnelle Strömung aufnimmt, abbremst und letztlich in nutzbaren Druck umwandelt. Dabei ist eine stoßfreie Verzögerung vorteilhaft für den Druckaufbau.

Der Erfindung liegt die Aufgabe zugrunde, einen Radialventilator bereit zu stellen, dessen Strömung verbessert ist, um einen möglichst hohen Wirkungsgrad bei gleichzeitig verbesserter Akustik aufzuweisen. Dabei soll der benötigte Bauraum des Radialventilators möglichst gering sein.

Diese Aufgabe wird durch die Merkmalskombination gemäß Patentanspruch 1 gelöst.

Erfindungsgemäß wird ein Radialventilator mit einem Motor und einem Ventilatorgehäuse vorgeschlagen, das mit einem Außenteil und einem Innenteil einen spiralförmigen Druckraum bildet. An dem Außenteil ist ein Druckstutzen angeordnet, der einen Ausblas des Radialventilators bildet. In dem Ventilatorgehäuse ist ein auf einer mit dem Motor verbundenen Welle angeordnetes Ventilatorrad vorgesehen, das im Betrieb Luft axial ansaugt und radial ausbläst. Radial an das Ventilatorrad angrenzend ist ein ringförmiger das Ventilatorrad umschließender Strömungsteiler angeordnet, der zusammen mit dem Ventilatorgehäuse um das Ventilatorrad einen Diffusor bildet, der unmittelbar in den Druckraum übergeht.

Für das Ventilatorrad ist es wichtig, dass es am Austritt über den gesamten Umfang einen gleichmäßigen Gegendruck hat. So leistet jede Schaufel des Ventilatorrads einen optimalen Beitrag zur Strömung. Eine ungleichförmige Druckverteilung rund um das Ventilatorrad würde zu negativen und unerwünschten Strömungsabrissen und Rückströmungen in das Ventilatorrad führen. Für den vorteilhaften Druckaufbau von dem Ventilatorrad in den Druckraum wird der Strömungsteiler um das Ventilatorrad vorgesehen, der zusammen mit dem Ventilatorgehäuse für die aus dem Ventilatorrad austretende Strömung einen Diffusor bereitstellt, der in unmittelbarer Strömungsverbindung mit dem Druckraum steht und für das Ventilatorrad den gleichmäßigen Gegendruck bereitstellt. Die radial ausgestoßene Luft durchströmt den Diffusor von radial innen nach radial außen bis zu dessen radialen Ende und tritt dann in den Druckraum ein.

Der Eintritt in den Druckraum liegt naturgemäß radial angrenzend an den Diffusor, der spiralförmige Druckraum selbst ist jedoch vorzugsweise axial angrenzend zu dem Diffusor in einer benachbarten Axialebene ausgebildet, so dass die Strömung von dem Strömungsteiler tangential in den Druckraum einströmt. Der Strömungsteiler kann sich weit nach radial außen erstrecken, so dass die Einströmung im radialen Außenbereich des Ventilatorgehäuses und dessen spiralförmigen Druckraums erfolgt und dabei einen definierten Korkenzieherwirbel erzeugt. Dadurch können Turbulenzverluste im spiralförmigen Druckraum vermindert werden.

Eine vorteilhafte Ausführungsform des Radialventilators sieht vor, dass das Au-ßenteil einen den Druckraum begrenzenden konstanten Außendurchmesser aufweist und eine Spiralform des Druckraums ausschließlich durch das Innenteil bestimmt ist. Der Bauraum des Radialventilators ist bei dieser Ausführung besonders kompakt. Durch die Nutzung des Strömungsteilers können die sich durch die über das Innenteil erzeugte Spirale des Druckraums ergebenden Nachteile ausgeglichen werden.

Ferner ist eine Ausführung des Radialventilators bevorzugt, bei welcher der spiralförmige Druckraum durch eine axiale und radiale Erweiterung gebildet ist. Durch die Vergrößerung des Druckraums in zwei Richtungen kann die Spiralform bei vergleichsweise geringem Raumbedarf realisiert werden.

Der Strömungsteiler steht bei dem Radialventilator vorzugsweise gegenüber einer radialen Innenwandfläche des Innenteils in radialer Richtung hervor und bildet somit eine Axialfläche des Druckraums. Der spiralförmige Druckraum liegt axial angrenzend an den Diffusor, wobei der Strömungsteiler vorteilhafterweise auf einer ersten axialen Seite eine Strömungsfläche für den Diffusor und auf einer gegenüberliegenden axialen Fläche eine axiale Wandfläche für den spiralförmigen Druckraum bildet.

Bei dem Radialventilator wird in einer Ausführungsvariante vorgesehen, dass der Motor eine Leiterplatte mit Elektronikbauteilen aufweist und das Innenteil den Druckraum gegenüber der Leiterplatte trennt. Somit ergibt sich eine axial geschachtelte kompakte Anordnung von Druckraum und Motorelektronik.

Erfindungsgemäß ist der Motor als Spalttopfmotor mit einem axial offenen Lagerrohr ausgebildet. Dabei ist erfindungsgemäß vorteilhaft vorgesehen, das Lagerrohr und das Innenteil einstückig auszubilden. Es können somit Funktionen des Motors mit denen der Aerodynamik zur Strömungslenkung kombiniert werden, ohne dass weitere Teile benötigt werden.

Der Radialventilator ist in einer Weiterbildung ferner dadurch gekennzeichnet, dass der Strömungsteiler integraler Bestandteil des Lagerrohres und der Rotorbaugruppe ist. In einer günstigen Ausführung ist der Strömungsteiler in das axial offene Lagerrohr eingesteckt und übernimmt zumindest abschnittsweise die Form des Innenteils.

Der Strömungsteiler weist in einer Ausführungsform eine topfartige axiale Einsenkung mit einem Durchmesser auf, der im Wesentlichen einem Außendurchmesser des Ventilatorrades entspricht. Das Ventilatorrad ist mit einem möglichst geringen Spaltmaß von 0,2 - 0,5mm zur Gewährleistung der Rotation abschnittsweise axial in die Einsenkung eingesetzt, so dass die von dem Ventilatorrad ausgeblasene Strömung unmittelbar von dem sich radial anschließenden Abschnitt des Strömungsteilers, der den Diffusor bildet, beeinflusst wird. Die Einsenkung ermöglicht einen axial kompakten Aufbau von Ventilatorrad und Strömungsteiler.

Der Radialventilator ist in einer Weiterbildung dadurch gekennzeichnet, dass der Strömungsteiler an seinem freien zum Druckraum weisenden Ende zumindest abschnittsweise eine Rundung aufweist. Insbesondere ist die Rundung an einem radialen Ende des Diffusors vorgesehen, um die tangentiale Einströmung in den Druckraum zu verbessern.

Als weitere Ausführungsvariante des Radialventilators weist das Innenteil an seinem radialen Außenrandabschnitt einen umlaufenden Axialvorsprung auf, der an dem Außenteil anliegt. Daraus ergibt sich eine axiale Einsenkung im Innenteil im Druckraum, wobei gleichzeitig über den umlaufenden Vorsprung eine ausreichende Anlagefläche zwischen Innenteil und Außenteil gewährleistet ist.

Ferner umfasst der Radialventilator in einer Ausführungsvariante mindestens ein Lager zur Lagerung der Welle, das zwischen dem Strömungsteiler und der Welle angeordnet ist. Vorzugsweise werden zwei axial beabstandete Lager vorgesehen, die über eine Feder verspannt und jeweils zwischen dem Strömungsteiler und der Welle angeordnet sind.

Bei dem Radialventilator ist der Motor vorzugsweise in einem Gehäusedeckel aufgenommen. Das Außenteil, das Innenteil und der Gehäusedeckel sind gegeneinander über Dichtungen abgedichtet, um Druckverluste zu minimieren.

Andere vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:
- Fig. 1: eine Draufsicht auf einen Radialventilator;
- Fig. 2: eine Schnittansicht A-A aus Fig. 1,
- Fig. 3: eine Schnittansicht B-B aus Fig. 1.

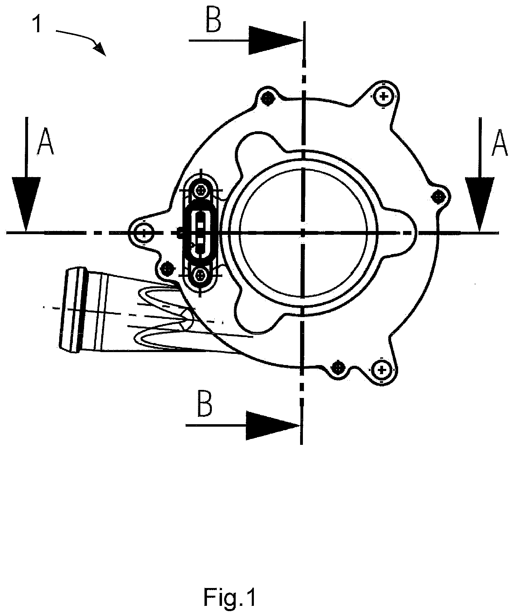

In den Figuren 1-3 ist ein Ausführungsbeispiel eines erfindungsgemäßen Radialventilators 1 in einer axialen Draufsicht und zwei Schnittansichten A-A und B-B dargestellt.

Der Radialventilator 1 umfasst einen als Spalttopfmotor ausgebildeten Elektromotor 2 mit einem Rotor 22 und einem Stator 32. Die Magneten des Rotors 22 sind an der Welle 7 befestigt, welche sich entlang der Rotationsachse axial durch den Radialventialtor 1 erstreckt. An der Welle 7 ist das als Radialventilatorrad ausgebildete Ventilatorrad 3 befestigt, welches im Betrieb über seine Laufradschaufeln Luft axial über den Einlass 69 ansaugt und über den Druckstutzen 33 am Ausblas 44 ausbläst. Ferner umfasst der Radialventilator 1 das Ventilatorgehäuse, welches gebildet wird durch das Außenteil 4, das Innenteil 5 und den Gehäusedeckel 17, in dem der Motor 2 aufgenommen ist. Axial zwischen dem Innenteil 5 und dem Motor 2 ist die Leiterplatte 10 mit den darauf fixierten Elektronikbauteilen 98 zur Regelung des Radialventilators 1 an dem Gehäusedeckel 17 befestigt. Das Innenteil 5 bildet einerseits einen Freiraum für die Elektronikbauteile 98 auf einer dem Motor 2 zuweisenden Seite, andererseits bestimmt es zusammen mit dem Außenteil den spiralförmigen Druckraum D auf der zum Außenteil 4 weisenden gegenüberliegenden axialen Seite des Radialventilators 1. Dabei erstreckt sich das Innenteil 5 nach radial außen bis zwischen das Außenteil 4 und den Gehäusedeckel 17 und wird über das Au-ßenteil 4 und den Gehäusedeckel 17 fixiert. Dichtungen 25 sind vorgesehen, um die beiden durch das Innenteil 5 getrennten axialen Bereiche abzudichten. Die Elektronikbauteile 98 sind im Freiraum angrenzend zu dem Druckraum D und mithin der Strömung zugewandt angeordnet, wodurch eine Wärmeabgabe an das Innenteil 5 und somit eine Kühlung erfolgen.

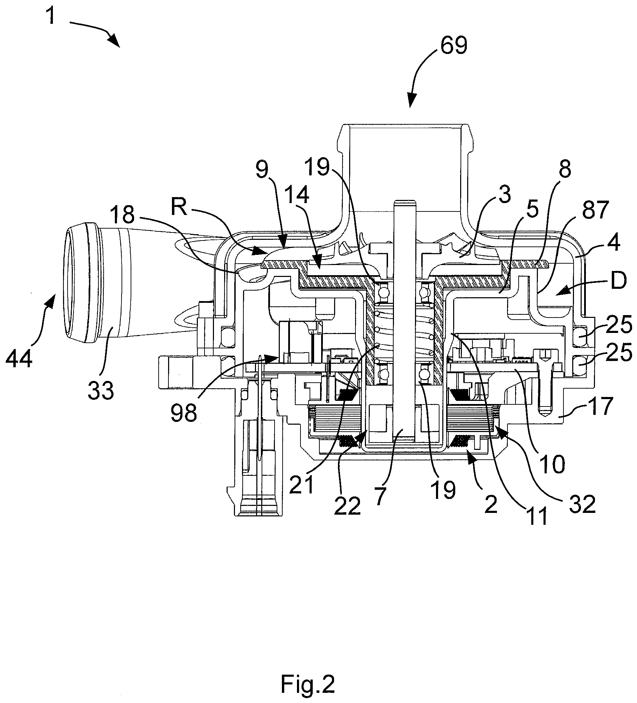

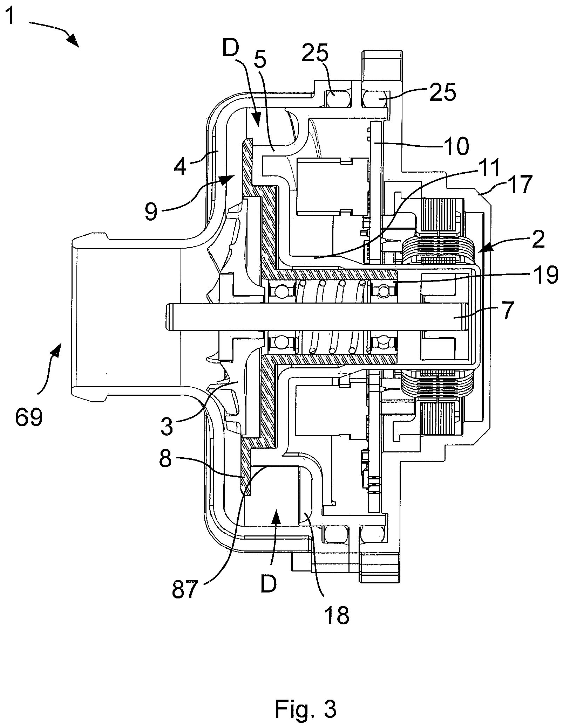

Radial an das Ventilatorrad 3 angrenzend ist der ringförmige, das Ventilatorrad 3 umschließende Strömungsteiler 8 angeordnet, der zusammen mit der Innenwandfläche des Außenteils 4 des Ventilatorgehäuses um das Ventilatorrad 3 den Diffusor 9 bildet. Die Innenwandfläche des Außenteils 4 und der Strömungsteiler 8 verlaufen im Bereich des Diffusors 9 senkrecht zur Rotationsachse nach radial außen. In einer alternativen Ausführung können die zueinander weisenden Wandflächen des Strömungsteilers und des Außenteils auch konvergieren. Das freie Ende des Strömungsteilers 8 bildet das Ende des Diffusors 9 und weist eine Rundung R auf. Der Diffusor 9 geht unmittelbar in den Druckraum D über. Die Spiralform des Druckraums D wird ausschließlich über das Innenteil 5 gebildet, das Außenteil 4 hat dabei einen konstanten Durchmesser. Unter Bezugnahme auf die Figuren 2 und 3 ist gut zu erkennen, dass sich der Druckraum D durch die Formgebung des Innenteils 5 sowohl axial als auch radial erweitert. Der Strömungsteiler 8 steht gegenüber der radialen Innenwandfläche 87 des Innenteils 5 in radialer Richtung hervor und bildet teilweise eine oberseitige Axialfläche des Druckraums D. In einer alternativen Ausführung schließt der Strömungsteiler umfänglich teilweise bündig mit der Innenwandfläche ab. Unterseitig weist das Innenteil 5 eine umlaufende kanalförmige Vertiefung auf, die radial außenseitig in einen den axialen Vorsprung 18 übergeht, der eine Anlagefläche zur Innenwandfläche des Außenteils 4 bildet. Der Druckraum D ist axial versetzt, jedoch unmittelbar angrenzend zu dem Diffusor 9 ausgebildet, so dass die von dem Ventilatorrad 3 erzeugte Strömung tangential aus dem Diffusor 9 in den Druckraum D strömt. Der Druckstutzen 33 ist an dem Außenteil 4 in Verlängerung des Druckraums D ausgebildet.

Achszentral erstreckt sich durch die Leiterplatte 10 das Lagerrohr 11 und nimmt den Rotor 22 auf. Das Lagerrohr 11 wird durch das Innenteil 5 einstückig mitgebildet. Der Strömungsteiler 8 ist als Einsatz ausgebildet und in das Lagerrohr 11 eingesteckt. Er bildet somit einen integralen Bestandteil des Lagerrohrs 11 und mithin der Rotorbaugruppe. Über zwei Lager 19 ist die Welle 7 an dem Strömungsteiler 8 und dem Lagerrohr 11 gelagert. Die beiden Lager 19 sind über die Feder 21 in axialer Richtung verspannt. Alternativ kann die Lagerung der Welle 7 auch gegenüber dem Lagerrohr 11 erfolgen.

Auf der Seite des Ventilatorrades 3 weist der Strömungsteiler 8 eine topfartige axiale Einsenkung 14 auf, in welche das Ventilatorrad 3 mit seiner Bodenscheibe eingesetzt ist, so dass der Austritt des Ventilatorrades 3 und die Oberfläche des angrenzenden Strömungsteilers 8 bündig in einer Axialebene liegen. Der Durchmesser der Einsenkung 14 ist gleich dem Außendurchmesser der Bodenscheibe des Ventilatorrades 3, so dass ein im Wesentlichen spaltfreier Übergang von dem Ventilatorrad 3 zu dem Strömungsteiler 8 erfolgt. Im Wesentlichen spaltfrei bedeutet dabei, dass die Rotation des Ventilatorrads 3 gegenüber dem Strömungsteiler 8 gewährleistet ist.

## Patentansprüche

1. Radialventilator (1) mit einem Motor (2) und einem Ventilatorgehäuse, das mit einem Außenteil (4) und einem Innenteil (5) einen spiralförmigen Druckraum (D) bildet, wobei an dem Außenteil ein einen Ausblas (44) des Radialventilators (1) bildender Druckstutzen (33) angeordnet ist, wobei in dem Ventilatorgehäuse ein auf einer mit dem Motor (2) verbundenen Welle (7) angeordnetes Ventilatorrad (3) vorgesehen ist, und wobei radial an das Ventilatorrad (3) angrenzend ein ringförmiger das Ventilatorrad (3) umschließender Strömungsteiler (8) angeordnet ist, der zusammen mit dem Ventilatorgehäuse um das Ventilatorrad (3) einen Diffusor (9) bildet, der unmittelbar in den Druckraum (D) übergeht, wobei der Motor (2) als Spalttopfmotor mit einem axial zumindest einseitig offenen Lagerrohr (11) ausgebildet ist, und wobei das Lagerrohr (11) und das Innenteil (5) einstückig ausgebildet sind.

2. Radialventilator nach Anspruch 1, **dadurch gekennzeichnet, dass** das Außenteil einen den Druckraum (D) begrenzenden konstanten Außendurchmesser aufweist und eine Spiralform des Druckraums (D) ausschließlich durch das Innenteil (5) bestimmt ist.

3. Radialventilator nach Anspruch 1 oder 2, **dadurch gekennzeichnet, dass** der spiralförmige Druckraum (D) durch eine axiale und radiale Erweiterung gebildet ist.

4. Radialventilator nach einem der vorigen Ansprüche, **dadurch gekennzeichnet, dass** der Strömungsteiler (8) gegenüber einer radialen Innenwandfläche des Innenteils (5) in radialer Richtung wenigstens bereichsweise hervorsteht und somit eine Axialfläche des Druckraums (D) bildet.

5. Radialventilator nach einem der vorigen Ansprüche, **dadurch gekennzeichnet, dass** der Motor (2) eine Leiterplatte (10) mit Elektronikbauteilen aufweist und das Innenteil (5) den Druckraum (D) gegenüber der Leiterplatte (10) trennt.

6. Radialventilator nach einem der vorigen Ansprüche, **dadurch gekennzeichnet, dass** der Strömungsteiler (8) integraler Bestandteil des Lagerrohres (11) ist.

7. Radialventilator nach einem der vorigen Ansprüche, **dadurch gekennzeichnet, dass** der Strömungsteiler (8) eine topfartige axiale Einsenkung (14) aufweist, die einen Durchmesser aufweist, der im Wesentlichen einem Außendurchmesser des Ventilatorrades (3) entspricht, wobei das Ventilatorrad (3) abschnittsweise axial in die Einsenkung (14) eingesetzt ist.

8. Radialventilator nach einem der vorigen Ansprüche, **dadurch gekennzeichnet, dass** der Strömungsteiler (8) in das axial offene Lagerrohr (11) eingesteckt ist.

9. Radialventilator nach dem vorigen Anspruch, ferner umfassend mindestens ein Lager (19) zur Lagerung der Welle (7), wobei das mindestens eine Lager (19) zwischen dem Strömungsteiler (8) und der Welle (7) angeordnet ist.

10. Radialventilator nach einem der vorigen Ansprüche, **dadurch gekennzeichnet, dass** der Strömungsteiler (8) an seinem freien zum Druckraum (D) weisenden Ende zumindest abschnittsweise eine Rundung (R) aufweist.

11. Radialventilator nach einem der vorigen Ansprüche, **dadurch gekennzeichnet, dass** das Innenteil (5) an seinem radialen Außenrandabschnitt einen umlaufenden Axialvorsprung (18) aufweist, der an dem Außenteil (4) anliegt.

12. Radialventilator nach einem der vorigen Ansprüche, **dadurch gekennzeichnet, dass** der Motor (2) in einem Gehäusedeckel (17) aufgenommen ist, wobei das Außenteil (4), das Innenteil (5) und der Gehäusedeckel (17) gegeneinander über Dichtungen (25) abgedichtet sind.

## Claims

1. A radial fan (1) with a motor (2) and a fan housing, an outer part (4) and an inner part (5) of which form a spiral-like pressure chamber (D), wherein a pressure connector (33) which forms an outlet (44) of the radial fan (1) is arranged on the outer part, wherein the fan housing is equipped with a fan wheel (3) which is arranged on a shaft (7) connected to the motor (2), and wherein an annular flow divider (8) which surrounds the fan wheel (3) is arranged adjacently to the fan wheel (3) in a radial direction, which flow divider together with the fan housing forms a diffuser (9) about the fan wheel (3), which diffuser (9) transitions directly into the pressure chamber (D), wherein the motor (2) is formed as a canned motor with a bearing tube (11) which is open axially on at least one side, and wherein the bearing tube (11) and the inner part (5) are formed as a single piece.

2. The radial fan according to claim 1, **characterized in that** the outer part has a constant outer diameter adjacent the pressure chamber (D), and a spiral shape of the pressure chamber (D) is determined exclusively by the inner part (5).

3. The radial fan according to claim 1 or 2, **characterized in that** the spiral-like pressure chamber (D) is formed by an axial and radial extension.

4. The radial fan according to any of the preceding claims, **characterized in that** the flow divider (8) protrudes, at least in sections, opposite a radial inner wall surface of the inner part (5) in the radial direction and thus forms an axial surface of the pressure chamber (D).

5. The radial fan according to any of the preceding claims, **characterized in that** the motor (2) has a circuit board (10) with electronic components and the inner part (5) separates the pressure chamber (D) as relates to the circuit board (10).

6. The radial fan according to any of the preceding claims, **characterized in that** the flow divider (8) is an integral component of the bearing tube (11).

7. The radial fan according to any of the preceding claims, **characterized in that** the flow divider (8) has a pot-like axial depression (14), which has a diameter which substantially corresponds to an outer diameter of the fan wheel (3), wherein the fan wheel (3) is inserted into the depression (14) axially in sections.

8. The radial fan according to any of the preceding claims, **characterized in that** the flow divider (8) is inserted into the axially open bearing tube (11).

9. The radial fan according to the preceding claim, further comprising at least one bearing (19) for supporting the shaft (7), wherein the at least one bearing (19) is arranged between the flow divider (8) and the shaft (7).

10. The radial fan according to any of the preceding claims, **characterized in that** the flow divider (8) has a curvature (R), at least in sections, on its free end as relates to the pressure chamber (D).

11. The radial fan according to any of the preceding claims, **characterized in that** the inner part (5) has a circumferential axial protrusion (18), which adjoins the outer part (4), on its radial outer edge section.

12. The radial fan according to any of the preceding claims, **characterized in that** the motor (2) is accommodated in a housing cover (17), wherein the outer part (4), the inner part (5), and the housing cover (17) are sealed off from one another using seals (25).

## Revendications

1. Ventilateur radial (1), comprenant un moteur (2) et un boîtier de ventilateur qui forme avec une partie extérieure (4) et une partie intérieure (5) une chambre de pression en forme de spirale (D), dans lequel une tubulure de refoulement (33) formant un dispositif de soufflage (44) du ventilateur radial (1) est disposée sur la partie extérieure, dans lequel une roue de ventilateur (3) disposée sur un arbre (7) relié au moteur (2) est prévue dans le boîtier de ventilateur, et dans lequel, de manière radialement adjacente à la roue de ventilateur (3), un diviseur de débit annulaire (8) entourant la roue de ventilateur (3) est disposé qui forme conjointement avec le boîtier de ventilateur autour de la roue de ventilateur (3) un diffuseur (9) qui se prolonge directement par la chambre de pression (D), dans lequel le moteur (2) est réalisé sous la forme d'un moteur à chemise d'entrefer muni d'un tube de palier (11) axialement ouvert au moins d'un côté, et dans lequel le tube de palier (11) et la partie intérieure (5) sont réalisés d'un seul tenant.

2. Ventilateur radial selon la revendication 1, **caractérisé en ce que** la partie extérieure présente un diamètre extérieur constant délimitant la chambre de pression (D), et une forme de spirale de la chambre de pression (D) est déterminée exclusivement par la partie intérieure (5).

3. Ventilateur radial selon la revendication 1 ou 2, **caractérisé en ce que** la chambre de pression en forme de spirale (D) est formée par un élargissement axial et radial.

4. Ventilateur radial selon l'une quelconque des revendications précédentes, **caractérisé en ce que** le diviseur de débit (8) fait au moins par endroits saillie par rapport à une surface de paroi intérieure radiale de la partie intérieure (5) dans la direction radiale et forme ainsi une surface axiale de la chambre de pression (D).

5. Ventilateur radial selon l'une quelconque des revendications précédentes, **caractérisé en ce que** le moteur (2) présente une carte de circuits imprimés (10) munie de composants électroniques, et la partie intérieure (5) isole la chambre de pression (D) par rapport à la carte de circuits imprimés.

6. Ventilateur radial selon l'une quelconque des revendications précédentes, **caractérisé en ce que** le diviseur de débit (8) fait partie intégrante du tube de palier (11).

7. Ventilateur radial selon l'une quelconque des revendications précédentes, **caractérisé en ce que** le diviseur de débit (8) présente un enfoncement axial (14) en forme de pot qui présente un diamètre qui correspond substantiellement à un diamètre extérieur de la roue de ventilateur (3), dans lequel la roue de ventilateur (3) est insérée par endroits axialement dans l'enfoncement (14).

8. Ventilateur radial selon l'une quelconque des revendications précédentes, **caractérisé en ce que** le diviseur de débit (8) est emboîté dans le tube de palier (11) axialement ouvert.

9. Ventilateur radial selon la revendication précédente, comprenant en outre au moins un palier (19) pour le montage de l'arbre (7), dans lequel ledit au moins un palier (19) est disposé entre le diviseur de débit (8) et l'arbre (7).

10. Ventilateur radial selon l'une quelconque des revendications précédentes, **caractérisé en ce que** le diviseur de débit (8) présente un arrondi (R) au moins par endroits à son extrémité libre orientée vers la chambre de pression (D).

11. Ventilateur radial selon l'une quelconque des revendications précédentes, **caractérisé en ce que** la partie intérieure (5) présente au niveau de sa portion marginale extérieure radiale une saillie axiale périphérique (18) qui est adjacente à la partie extérieure (4).

12. Ventilateur radial selon l'une quelconque des revendications précédentes, **caractérisé en ce que** le moteur (2) est reçu dans un couvercle de boîtier (17), dans lequel la partie extérieure (4), la partie intérieure (5) et le couvercle de boîtier (17) sont rendus étanches les uns par rapport aux autres par des joints d'étanchéité (25).
